(19) Europäisches Patentamt
European Patent Office
Office européen des brevets

(11) **EP 4 432 293 A1**

(12) **EUROPEAN PATENT APPLICATION**

(43) Date of publication:
**18.09.2024 Bulletin 2024/38**

(21) Application number: **23161477.7**

(22) Date of filing: **13.03.2023**

(51) International Patent Classification (IPC):
*G16C 20/30* (2019.01)    *G16C 20/64* (2019.01)

(52) Cooperative Patent Classification (CPC):
**G16C 20/64;** G16C 20/30; G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fraunhofer-Gesellschaft zur Förderung der angewandten Forschung e.V.**
**80686 München (DE)**

(72) Inventors:
• **Dr. BAUER, Thilo**
**91301 Forchheim (DE)**
• **SINGH, Satnam**
**85354 Freising (DE)**

(74) Representative: **Maikowski & Ninnemann**
**Patentanwälte Partnerschaft mbB**
**Postfach 15 09 20**
**10671 Berlin (DE)**

(54) **METHOD FOR SCREENING SUBSTANCES HAVING A REQUIRED PHYSICAL PROPERTY, CHEMICAL PROPERTY OR PHYSIOLOGICAL EFFECT FROM A GROUP OF SUBSTANCES USING ELECTRON DENSITIES**

(57)    The present invention provides a method for screening substances having a requested physical property, chemical property or physiological effect from a group of substances comprising the steps
• providing a group of $k$ substances by a user, wherein $k \in N$;
• providing a classification or a property label for a physical property, chemical property or physiological effect comprising $C_i$ classes or $V_i$ values, wherein $i \in N$;
• calculating electron density cube files for every substance by semiempirical molecular calculation and providing weights $W_k$, such as electronegativity or electron affinity cube files for every substance;
• providing a trained artificial neural network architecture for the classification or label property, wherein the artificial neural network architecture uses the electron density cube files and weights $W_k$ as input to calculate a tensor of shape of each substance describing the spatial structure of the substance and a tensor of weights, such as electronegativity or electron affinity assigned to the spatial structure of the substance;
• assigning each substance to a class $C_i$ or a value $V_i$ by utilizing the tensor of weights, such as electronegativity or electron affinity of substance k as weighting for the electronic density cube files in the artificial neural network architecture;
• displaying and/or outputting of the substances assigned to the classes $C_i$ of the classification or assigned to the values $V_i$ of the property label;
• selecting the substances assigned to the class $C_i$ with the required physical property, chemical property or physiological effect or assigned to the required value Vi of a physical property, chemical property or physiological effect;
• experimental verification by a user of the physical property, chemical property or physiological effect of at least part of the selected substances.

EP 4 432 293 A1

**Description**

The present invention provides a method for screening substances having a requested physical property, chemical property or physiological effect from a group of substances comprising the steps

- providing a group of $k$ substances by a user, wherein $k \in N$;
- providing a classification or a property label for a physical property, chemical property or physiological effect comprising $C_i$ classes or $V_i$ values, wherein $i \in N$;
- calculating electron density cube files for every substance by semiempirical molecular calculation and providing weights $W_k$, such as electronegativity or electron affinity cube files for every substance;
- providing a trained artificial neural network architecture for the classification or label property, wherein the artificial neural network architecture uses the electron density cube files and weights $W_k$ as input to calculate a tensor of shape of each substance describing the spatial structure of the substance and a tensor of weights, such as electronegativity or electron affinity assigned to the spatial structure of the substance;
- assigning each substance to a class $C_i$ or a value $V_i$ by utilizing the tensor of weights, such as electronegativity or electron affinity of substance k as weighting for the electronic density cube files in the artificial neural network architecture;
- displaying and/or outputting of the substances assigned to the classes $C_i$ of the classification or assigned to the values $V_i$ of the property label;
- selecting the substances assigned to the class $C_i$ with the required physical property, chemical property or physiological effect or assigned to the required value Vi of a physical property, chemical property or physiological effect;
- experimental verification by a user of the physical property, chemical property or physiological effect of at least part of the selected substances.

Chemical substances have chemical properties, physical properties and physiological effects. While physical properties can be quantified by measuring underlying physical quantities, chemical properties can be quantified by measuring an underlying chemical quantity when a substance interacts with another substance. The physical properties of a substance include, for example, boiling point, melting point, volatility and the colour of the substance. The solubility in water, on the other hand, is counted among the chemical properties of a substance.

Furthermore, chemical substances can have effects on the physiology of organisms (physiological effects). This includes physical and chemical substance properties under the aspect of perceptibility or the effect on an organism. Examples are the smell and the taste of a substance.

Chemical properties, physical properties and physiological effects are of great interest for a variety of applications. According to the invention, this includes properties such as taste or odour of substances. Physiological effects are induced by properties of substances that have effects on the organism of living beings. Furthermore, according to the invention, this also includes the classification of substances into permitted and non-permitted chemicals in cosmetics and personal care. This is regulated by the use authorization according to Articles Regulation, Annex II - Restricted Substances the Annex II of the European Chemicals Agency (ECHA). The taste of substances for example directly appeals to the human sense of taste and thus decisively influences the eating behavior of humans and in particular which foods are perceived as pleasant or also as unpleasant. The taste produced by substances is therefore of great importance, especially in the food industry.

Smell is one of the five human senses and plays an important role in daily life. For example, the smell of food influences our eating behavior [1] and smells influence human memory in various evolutionary situations [2]. In addition to the importance of odours for humans, they also play an important role in the economy, especially in the food and cosmetics industries, where the development of new flavours and the identification of odour-active substances are essential. In the development of new odourants, a predictive approach during molecular design is required to reduce the space of candidate substances from virtually all to a promising set of structures.

In the state of the art, sensory-trained experts in industry and science have to smell substances to determine their odour. Due to largely unknown structure-odour relationships, the principle of trial-and-error prevails in the development of aroma substances or the identification of odour-active substances. This is very time-consuming, personnel-intensive, and therefore uneconomical.

As a result, it is desirable to be able to deduce physical properties, chemical properties or physiological effects of a substance from its structure.

Machine learning models have already been used to predict certain properties. Traditionally, substances have been represented using InChI [3] (International Chemical Identifier) or SMILES [4] (Simplified Molecular Input Line Entry Specification) notation when used in machine learning tasks to predict properties of substances or to even generate new structures. Another method of encoding molecular structure and features is the SMARTS [5] (SMILES ARbitrary Target Specification) notation. These rule-based methods have the benefit of being rather straightforward to generate and being easy to understand by chemists. Such representations have been used extensively with machine learning in previous works such as those to generate a new molecular structure [6-16]. Additionally, other encoding schemes such as molecular graphs have been widely combined with machine learning [8, 17-21] to predict molecular properties [22, 23] such as toxicity [24, 25] or to predict odour of substances [26-30] and even in drugs discovery [31-34] among other applications.

However, such a 2D representation of chemical substances that are essentially three-dimensional signals does not capture their spatial domain. The SMILES notation, for example, does not capture the similarity of molecules, so similar chemical substances may be encoded differently using very different SMILES strings and there's no standard method to generate a canonical representation [35, 36]. Additionally, there are molecules that cannot be defined by a graph model like those having delocalized bonds such as metal carbonyl complexes [35].

Based on the prior art, it is therefore the object of the invention to provide a method by which substances with a required physical property, chemical property or physiological effect can be selected and or generated from a predetermined set of substances without having to examine all the substances with respect to the desired property or effect by experimental methods.

This purpose is addressed by the present invention by a method according to claim 1.

## Detailed Description

The invention provides a method for screening substances having a requested physical property, chemical property or physiological effect from a group of substances.

According to the invention a group of $k$ chemical substances is provided by a user, wherein $k \in N$. In one embodiment a group of $k$ molecules is provided. In one embodiment of the present invention, between 20 and 5000 substances are provided, preferably between 100 and 4000 substances are provided, most preferably between 500 and 3000 substances are provided. In this case, provided means first of all that the structural formulae of the chemical substances are available and thus provided. This is possible, for example, by providing the chemical substances in the structural code SMILES, which encodes structural patterns as SMARTS [37-39]. In addition, however, it is possible to have each of the chemical substances available physically present at a later time for experimental confirmation.

Further a classification or a property label is provided according to a chemical property, physical property and/or physiological effect of a chemical substance comprising $C_i$ classes or $V_i$ values, where $i \in N$.

If a classification is given, in one embodiment of the invention, the classification is selected from structure-based properties of substances, in particular from the group comprising odour, taste, colour, toxicity, volatility, water solubility and permitted and non-permitted chemicals in cosmetics and personal care.

A classification comprises several classes, for example, classification of water solubility comprises classes hydrophilic and hydrophobic or the classification of the volatility of a compound in high, medium or low volatility classes The classification of permitted and non-permitted chemicals in cosmetics and personal care comprises these very classes, namely permitted chemicals in cosmetics and personal care and non-permitted chemicals in cosmetics and personal care. The classification of toxicity comprises classes toxic and non-toxic. The classification of colour may include different colours as classes, for example blue, red, yellow, green. The classification of taste corresponds to different tastes, such as bitter, sour, sweet, salty and umami. The odour classification preferably comprises odour directions such as, but not limited to, 'woody, resinous', 'floral', 'fruity, not citric', 'medicinal', 'perfumed', 'light', 'heavy', 'sweet', 'aromatic', 'fragrant', 'disgusting', 'odorless' as classes.

If a property label is given the property label comprises $V_i$ continuous values. Property labels can be for example boiling point, melting point, solubility, aroma thresholds, partition coefficient and so on.

Further, electron density cube files for every substance are calculated by semiempirical molecular calculation and weights $W_k$, such as electronegativity or electron affinity cube files are provided for every substance. In one embodiment weights $W_k$, such as electronegativity or electron affinity cube files are calculated as well by semiempirical molecular calculation for each of the $k$ substances.

In a preferred embodiment of the invention, the semiempirical molecular calculation is done in the following way. First of all, the canonical SMILES representation is used as a starting point for each of the k substances. These SMILES are converted to a mol2 format and optimized by using Merck Molecular Force Field (MMFF) [40] method in rdkit [41] and saved to disk as *inp* files. These files are then passed into EMPIRE [42] software for semi-empirical molecular calculations. For this purpose, the AM1S [43] Hamiltonian is chosen and a full geometric optimization in the Cartesian coordinates is performed after centering the molecular structure and orienting it according to its principal axis. As the output, the wavefunction of the selected molecular structure at its electronic ground state is received as a HDF5 wavefunction file, which is then used to generate electron density, electronegativity and electron-affinity cube files using the *eh5cube* software from Cepos [42].

In a further embodiment of the invention, alternative software such as GAUSSIAN [44] is used instead of EMPIRE. Further, alternative approximations instead of DFT or different types of geometric optimizations can be used.

Such alternatives are well known in the state of the art.

The final dataset consists of cube files comprising the electronic density distribution of the $k$ substances along with weights $W_k$, such as electronegativity and/or electron-affinity cube files for substance k. In general the cube files are N dimension tensors consisting of information related to the substances such as but not limited to their electron densities, electron affinity, electronegativities in 3D space.

Further a trained artificial neural network architecture for the classification or property label is provided, wherein the artificial neural network architecture uses the electron density cube files and electronegativity cube files or electron affinity cube files as input to calculate a tensor of shape of each substance describing the spatial structure of the substance and a tensor of electronegativity or a tensor of electron affinity assigned to the spatial structure of the substance.

In one embodiment, the artificial neural network architecture is a segmentation network or a Generative Adversarial Network (GAN). Both are open source networks and known to the person skilled in the art.

In a preferred embodiment the segmentation network is a modified 3D-UNet, modified VNet or modified Inception architecture, or similar networks.

The segmentation networks 3D-UNet, VNet and Inception architecture are known per se. Their modification will be described further below.

To assign each substance to a class $C_i$ or a label value $V_i$ the artificial neural network performs a calculation of the structure of each of the k substances.

To calculate the structure of each of the k substances, the generated electron density cube files and electronegativity cube files or electron affinity cube files for each substance are considered as a stack of images over an arbitrary dimension and calculating the structure of the substance by localizing atomic centers corresponds to the problem of assigning different atomic classes to the pixels of these stacked images, where most of the pixels belong to a so called "background class" creating sparsity, and contributing to a clear imbalance in the atomic classes is the abundance of elements in nature and in the datasets. The pixels of the stacked images are also called voxels in the following. Mostly the distribution of elements shows a clear imbalance towards elements that occur more frequently in nature such as hydrogen and carbon. These elements are then associated with an atomic class number $n$, where $n \in N$ and with atomic class 0 being assigned to the background voxels.

Further, for each of the $k$ substances a label for calculating its structure is created. Such a label is preferably a tensor of the same shape as the corresponding electronic density cube file. Then, the voxels corresponding to the atomic center of the element are assigned the value corresponding to its atomic class. All other voxels are set to zero. For example, voxel locations corresponding to element hydrogen were set to atomic class 1, those corresponding to carbon were set to atomic class 2 and likewise for all other relevant elements.

According to the invention each substance is assigned to a class $C_i$ of the classification or a value $V_i$ of a property label. Therefore, in one embodiment a local map of electronegativity is calculated for each of the k substances by assigning voxels with a certain electronegativity value to a certain electronegativity class. In a further embodiment of the invention a local map of electron affinity is calculated for each of the k substances by assigning voxels with a certain electron affinity value to a certain electron affinity class. The following steps are further described for electronegativity but apply as well for electron affinity.

Accordingly, in one embodiment a local map of electronegativity is calculated by assigning voxels with electronegativity values larger than the upper 90 percentile as electronegativity class 2 denoting high reactive sites, whereas voxels with electronegativity values less than the lower 10 percentile are assigned to the electronegativity class 1, denoting low reactive sites and all other voxels are allocated as electronegativity class 0, denoting a medium reactive region. Hence, these labels denote regions of high, medium, and low reactivity for each of the k substances. In a preferred embodiment, an electronegativity file is created in the same way as an electron density file. While creating the electron density files, EMPIRE creates some more files such as hardness, softness, electron affinity, electronegativity among others.

The local maps of electronegativity are used as labels which are input in the neural network architecture. Additionally, since for noninert atoms, high electronegativity is correlated to high reactivity [45], in one embodiment a ternary reactivity mask is derived using electronegativity cube files to create labels for this task.

In a further embodiment electronegativity is not encoded in three electronegativity classes but in two electronegativity classes.

Further, in one embodiment the electronic density cube file and their corresponding local electronegativity map is illustrated as a substance structure with an overlaid electronegativity map.

The electron densities included in the electron density cube files and the local map of electronegativity from selective sites of the substance can be used to infer properties of substance such as the classification of substances into classes $C_i$ or assigning a substance to a label value $V_i$ of physical properties, chemical properties or physiological effects. For this purpose, an adaptive max pooling layer and two fully connected layers are included in the artificial neural network architecture used for the previously described tasks. If a segmentation network such as a 3D-UNet, a VNet or an Inception architecture is basically used the adaptive max pooling layer and two fully connected layers are the modification or at least part of the modification of these segmentation networks. Accordingly, the method of the invention is based on the Density Functional Theory that states that knowing the electron density of a substance allows direct derivation of various properties such as electrostatic potentials, energies, or dipoles of a substance [46, 47].

The input electronic density is passed through the artificial neural network architecture comprising the adaptive max pooling layer and two fully connected layers to get the structure of the substance as described in a tensor of shape (N, C, B_x, B_y, B_z) along with the tensor of electronegativity which consists of three channels consisting of logits for each of the three classes of electronegativity, which are the raw predicted values from the final layers of the artificial neural network. The tensor of electronegativity is also called electronegativity logit mask in the following. Here, N is the batch size which is similar to the number of substances k; C is the number of elemental classes, corresponding to the different element types in the dataset; (B_x, B_y, B_z) corresponds to the shape of the batch cube, i.e. shape of the overall tensor made by padding all the cubes in a given batch.

Accordingly, the tensor of electronegativity comprises the spatial distribution of the electronegativity encoded in discrete electronegativity classes, preferably encoded in two or three electronegativity classes.

To classify a substance into a class $C_i$ or to assign a substance to a label value $V_i$ belonging to requested physical property, chemical property or physiological effect, the electronegativity logit mask is multiplied with the input electron densities, namely the electron density cube file, and pass the resulting tensor through the adaptive max pooling, batch normalization and fully connected layers. The idea behind this is that the logits of the electronegativity maps, which are comprised in the electronegativity logit mask, serve as weights for the electronic densities and following this with max pooling allows selection of the highest of those weighted densities from within the kernels, allowing to filter the reactive regions in the substance.

Assignment to class $C_i$ is done by using the output produced by the artificial neural network. If $C_i$ belongs to physical properties, the output is of the shape (N, C, B_x, B_y, B_z) and the assignment is performed by using a softmax activation with 0.5 threshold, where the assigned classes $C_i$ are present as value 1 and rest as zeros. For other properties, the output is of the shape (N, C) and a sigmoid activation with threshold of 0.5 is used for class assignment. The assigned class $C_i$ is present as 1 and the rest as zeros. The assignment is optimised by minimising a loss function between the actual assignment and the assignment from the artificial neural network, e.g. by minimising the Cross Entropy Loss, mean square error loss (MSE) or L1 error.

Assignment to value $V_i$ of a property label is also done by using the output produced by the artificial neural network. If $V_i$ belongs to physical properties, the output is of the shape (N, C, B_x, B_y, B_z) and the assignment is performed by using a softmax activation with 0.5 threshold, where the assigned values $V_i$ are present as value 1 and rest as zeros. For other properties, the output is of the shape (N, C) and a linear activation with threshold of 0.5 is used for assignment. The assigned values $V_i$ are present as continuous values. The assignment is optimised by minimising a loss function between the actual assignment and the assignment from the artificial neural network, e.g. by minimising the Cross Entropy Loss, mean square error loss (MSE) or L1 error.

According to the invention substances which are assigned to the classes $C_i$ or a value $V_i$ of the classification are displaying and/or outputting. Displaying can happen on a suitable device such as a display of a PC, tablet or such a like. Outputting can also be a print of the results.

Further, the substances assigned to the class $C_i$ or a value $V_i$ with the requested physical property, chemical property or physiological effect are selected and an experimental verification by a user of the physical property, chemical property or physiological effect of at least a part of the selected substances is performed.

The experimental verification simultaneously verifies the classification of the substance or the assignment to a value $V_i$ by a user. The type of experimental verification depends on the classification or property label used. The following table provides a non-exhaustive overview of common experimental methods that can be used to verify physical properties, chemical properties and physiological effects of substances. All other common experimental methods known to those skilled in the art are equally applicable.

| Classification or property label | Experimental verification method |
|---|---|
| taste | taste test by trained person |
| odour | odour test by trained person |
| water solubility | conductivity measurements to determine the solubility product, UV/Vis spectroscopy |
| color | spectroscopy |
| melting point | measured by heating the sample |

(continued)

| Classification or property label | Experimental verification method |
|---|---|
| boiling point | measured using boiling point equation Kb = RTb2M/ΔHv, R is that the universal gas constant. Tb is that the boiling temperature of the pure solvent [in Kelvin] M is that the molar mass of the solvent |
| partition coefficient | experimentally determined by octanol-water-equilibrium with thin layer chromatography or liquid chromatography |
| volatility | Experimentally determined by vapour pressure measurement using a manometer |

The present invention thus enables a significant saving in cost, manpower and technical effort, since it is no longer necessary to experimentally investigate all $k$ substances of a provided group in order to select at least one substance of a particular class $C_i$ or with a particular value $V_i$ and thus with a particular physical property, chemical property or physiological effect. By applying the inventive method, a selection of substances is made and the subsequent experimental verification can be carried out specifically with this selection of substances. This saves time and costs compared to state-of-the-art methods. Moreover, it is not necessary to have all substances available for experimental investigations, which saves additional costs.

Advantageously, the inventive method enables to take into account the spatial domain of the substances under investigation. Thus, the calculation becomes more precise compared to state of the art methods.

Training

According to the invention the artificial neural network architecture for the classification is trained for a defined classification by a training dataset, wherein the training dataset consists of substances with known assignment to the classes $C_i$ of the classification or with known assignment to a value $V_i$ of a property label.

The training of the artificial neural network is performed in two steps. First step is to investigate if the chosen artificial neural network architecture and the labeling approach can be used to learn meaningful information about the structure of a substance. As the second step, the second set of labels is input, namely, the electronegativity maps or electron affinity maps to jointly train the artificial neural network architecture to learn and predict the structure and the corresponding electronegativity maps or electron affinity maps simultaneously.

In a preferred embodiment the artificial neural network architecture is trained using PyTorch [50] library for Python. The hyper-parameters for both trainings are selected by performing a grid search over a common set of hyper-parameters.

The loss function for the classes of the structure is chosen as cross-entropy loss, mean square error loss (MSE) or L1 error between the actual voxels for a given class and the predicted locations for the same class and class weights can be used to counter the class imbalance. For the prediction of electronegativity maps or electron affinity maps, dice loss [51] is calculated between the ground truth maps and the predictions from the network and the loss function for joint training of the two tasks is the sum of the cross-entropy and dice loss. To evaluate the performance of the artificial neural network architecture, three different metrics can be used such as, L1 error, Ratio Of Prediction and dice coefficient between the ground truth and predictions.

Consider some substance $M$ with $n$ atomic centers defined by cartesian coordinates $(x,y,z)$ corresponding to

$$\begin{Bmatrix} (x_1, y_1, z_1) \\ (x_2, y_2, z_2) \\ ... \\ x_n, y_n, z_n \end{Bmatrix}$$

some class, say hydrogen as shown: . For this substance, and this class, let there be $m$ predictions

$$\begin{Bmatrix} (x'_1, y'_1, z'_1) \\ (x'_2, y'_2, z'_2) \\ ... \\ x'_m, y'_m, z'_m \end{Bmatrix}$$

shown as . The structure prediction task is to predict the $n$ positive spikes in a sparse 3D space consisting of mostly zeros.

It can be observed that in the calculation $n \neq m$ and $(x_i, y_i, z_i) \neq (x'_i, y'_i, z'_i)$ along one or more dimensions indicating over or under prediction along with errors in the exact atomic center voxel locations. Concretely, often not only the actual location for the atomic center is predicted by the calculation but their immediate neighbors are often falsely calculated as well.

Thus, quantifying the performance of the artificial neural network architecture using metrics such as simple accuracies by comparing exact locations does not give the full picture. For this reason, preferably three metrics that can capture the performance of the artificial neural network architecture are calculated.

Firstly, a metric called the Ratio Of Prediction (ROP) is calculated. ROP is defined as the ratio of the number of predicted voxels for a given atomic class to the actual number of voxels for that atomic class. This helps establishing a relationship between $n$ and $m$ from the example above. A value of $ROP > 1$ indicates overprediction of the number of atomic centers, i.e., $m > n$, whereas $ROP < 1$ indicates underprediction of the number of atomic centers, i.e., $m < n$. For example, if atomic class 1 (hydrogen) has 100 atoms in a batch of 20 substances, and the model predicts 90 voxels for that atomic class in the same batch, the ROP value is then 0.9 for the batch. The ROP values are summed up and then averaged across the batch size. For an ideal prediction, the ROP is expected to approach 1, indicating that the artificial neural network architecture calculates the correct number of atoms for a given atomic class. Additionally, the dice-coefficient between the predicted tensor and the ground truth tensor for each element class number $n$ is calculated. This quantifies the ability of the artificial network to segment the different atomic classes. Since it's possible that some atomic classes $n$ occur only in a small subset of a dataset, these dice values are calculated when there exists at least one voxel corresponding to that atomic class $n$ in a batch before being averaged over the batches that the atomic class $n$ occurs in.

Finally, the L1 error per atomic class $n$ between the predicted voxel locations against the actual voxel locations is calculated as well. This helps to quantify the error in the predicted locations of the atomic centers. The L1 error, however, will not allow differentiation between errors in the counts due to erroneously calculated voxels and those due to mislocated atomic center voxels, hence we use the three metrics in conjunction to evaluate the overall performance.

To evaluate the performance of the artificial neural network architecture in calculating the electronegativity maps or electron affinity maps, dice coefficients for each of the three or two electronegativity classes of the electronegativity maps or dice coefficients for each of the three or two electron affinity classes of the electron affinity maps can be calculated.

In one embodiment in the training data set there occurs a clear imbalance between substances belonging to different classes of a classification. For example, there are overall 855 substances from the 'Allowed/Non-Hazardous' class, and 501 substances from the 'Prohibited/Health Hazardous' class that can be also considered as toxic for cosmetic usage. In a preferred embodiment this class imbalance is combated by introducing a class weight in the binary cross entropy loss equal to the inverse ratio of occurrence of the two classes.

According to the invention 3D electronic densities are used as training data for an artificial neural network architecture to allow capturing of the spatial nature of substances. Additionally, the electron densities included in the electron density cube files and local electronegativity maps or electron affinity maps from selective sites of the substance are used to infer properties of substances such as the classification of substances into classes $C_i$ or assignment of a substance to a value $V_i$ of a property label of physical properties, chemical properties or physiological effects.

Thus, it is demonstrated that electronic densities can be used as training data for artificial neural network architectures to infer the structure of the underlying chemical substances. Additionally, the local electronegativity maps or local electron affinity maps of chemical compounds are segmented and can be used to identify sites of high and low electronegativity or electron affinity. These are commonly considered as active sites where reactions can take place and together with electron densities can be used in classification $C_i$ of compounds e.g. into chemical substances that are allowed in cosmetics i.e., are not hazardous and those that are health hazards and hence prohibited.

The overall problem of using molecular electronic densities to classify a substance as hazardous for cosmetic use can be divided into three subtasks. Firstly, the underlying substance itself is inferred, i.e., the artificial neural network predicts the type of element and localizes the atomic centers of the substance based on the cartesian coordinates of its atomic center extracted from the cube file. The invention serves as a proof of concept that an artificial neural network architecture can learn meaningful information using the electron densities. Secondly, the artificial neural network architecture can simultaneously also calculate the electronegativity maps or electron affinity maps for the given compound to predict regions of high, medium, and low reactivity and finally, this information is suitable to classify substances into classes $C_i$ of requested physical properties, chemical properties or physiological effects.

In the following the method of the invention is further described by 4 figures and 3 examples.

Figure 1     illustrates electronic density (A) and the corresponding electronegativity map (B) for the molecule CC(=O)OC1CC2CC1C3C2CCC3;

Figure 2     illustrates electronegativity regions for 3 molecules (A) from a test set along with their corresponding calculated segmentation (B);

Figure 3     (A) and (B) illustrate Confusion matrix for classification;

Figure 4     (A) illustrates a native 3D Unet and (B) a modified 3D Unet.

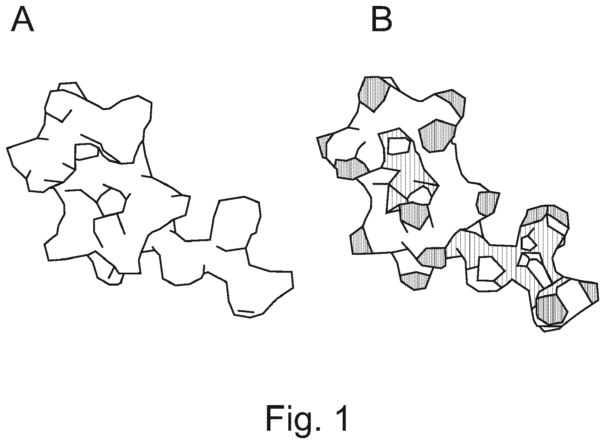

**Figure 1 to 3** are further explained in connection with the examples.

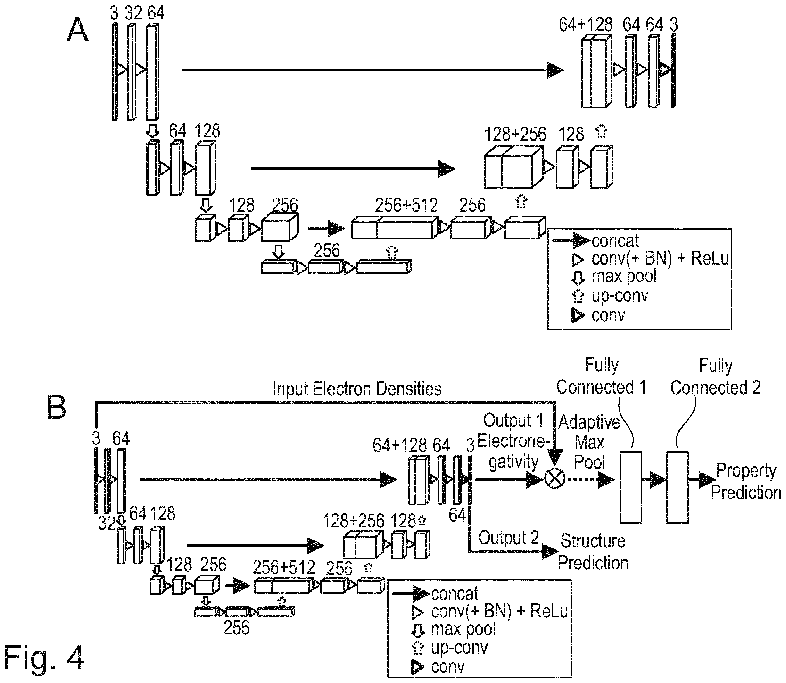

**Figure 4** (A) illustrates a native 3D Unet as it is known to persons skilled in the art. Figure 4 (B) illustrates a modified 3D Unet according to the invention. The additional MaxPool and the two additional fully connected layers are shown.

## Example 1 - calculation of structure and electron density

The structure and electron density for the molecule CC(=O)OC1CC2CC1C3C2CCC3 have been calculated by the method of the present invention.

Table 1: *Shows the distribution of elements in a set of k molecules which included the molecule CC(=O) OC1CC2CC1C3C2CCC3. Structure and electronegativity map were calculated according to the invention.*

| Atomic Number | Atomic Class Number $n$ | Element | % in dataset |
|---|---|---|---|
| 1 | 1 | H | 53.59 |
| 6 | 2 | C | 31.55 |
| 7 | 3 | N | 4.19 |
| 8 | 4 | O | 6.12 |
| 9 | 5 | F | 0.72 |
| 14 | 6 | Si | 0.75 |
| 15 | 7 | P | 0.73 |
| 16 | 8 | S | 0.86 |
| 17 | 9 | Cl | 0.75 |
| 35 | 10 | Br | 0.74 |

**Figure 1** illustrates the calculated electronic density (A) and the corresponding electronegativity map (B) for the molecule CC(=O)OC1CC2CC1C3C2CCC3. The electronegativity values have been overlaid on the molecular structure and then divided into 3 electronegativity classes. The shaded region in figure 1 (B) shows regions of high electronegativity and hence these voxels are marked as electronegativity class 2. Black regions with white stripes show regions of low electronegativity and these voxels are marked as electronegativity class 1. All other voxels are marked as electronegativity class 0 and shown as white.

## Example 2 - Training of artificial neural network architecture

Table 2 shows the metrics described for training the artificial neural network architecture to calculate the structure of a substance averaged over a test set. It is observed that for almost all atomic classes $n$, except for Sulphur (class 8), the artificial neural network architecture calculates more voxels than present in the ground truth. This is especially the case for class 6, silicon.

Table 2: *Three metrics were calculated for evaluating the performance of the artificial neural network architecture for structure prediction using electronic densities as input and centers of atoms as labels. The L1 error is considerably high for atomic class 6 (silicon). This is also seen in its low Dice coefficient score.*

| Atomic Number | Atomic Class Number $n$ | ROP | L1 error | Dice |
|---|---|---|---|---|
| - | 0 | 0.9983 | - | 0.9989 |
| 1 | 1 | 2.8504 | 35.2044 | 0.4670 |
| 6 | 2 | 6.2873 | 62.8729 | 0.2779 |
| 7 | 3 | 1.9807 | 3.1153 | 0.3363 |
| 8 | 4 | 2.9929 | 5.3812 | 0.5200 |
| 9 | 5 | 2.2628 | 13.7500 | 0.2701 |
| 14 | 6 | 19.3000 | 34.5000 | 0.0272 |

(continued)

| Atomic Number | Atomic Class Number $n$ | ROP | L1 error | Dice |
|---|---|---|---|---|
| 15 | 7 | 4.3333 | 4.0000 | 0.3846 |
| 16 | 8 | 0.6250 | 1.5000 | 0.1667 |
| 17 | 9 | 2.4340 | 4.5000 | 0.6585 |
| 35 | 10 | 2.8333 | 2.6667 | 0.5370 |
| ∅ | - | 4.2634 | 16.7490 | 0.4222 |

For this purpose, the average molecular weights and the average fraction of sp3 (fsp3) carbons [48,49] for all molecules belonging to all the different element types were compared to quantify the complexity of these elemental classes $n$. This is summarized in Table 3. It can be observed that molecules containing Si on average have a higher fsp3 count than other molecules and a comparatively high mean molecular weight. This could be indicative of higher complexity of these molecules causing the artificial neural network architecture to make false predictions for this particular class.

Table 3: The mean fraction of sp3 carbons and the mean molecular weight calculated for each element type present in the dataset. The high fsp3 and mean molecular weight suggests that the dataset samples containing silicon consists of more 'complicated' molecular structures than other classes.

| Element | Mean fsp3 | Mean Mol Wt |
|---|---|---|
| C | 0.6222 | 234.56 |
| N | 0.4715 | 245.51 |
| O | 0.6107 | 239.8 |
| F | 0.4016 | 337.51 |
| Si | 0.7594 | 322.39 |
| P | 0.7123 | 309.14 |
| S | 0.5361 | 274.24 |
| Cl | 0.4303 | 265.24 |
| Br | 0.5407 | 332.96 |

Table 4 shows the metrics described for the joint prediction of structure and electronegativity maps, namely, Ratio Of Prediction, L1 error, dice coefficient for structure prediction and the dice coefficient for segmentation of the electronegativity maps. Overall, there is a similar performance compared to the single structure learning task. For this machine learning task, again, silicon, atomic class 6 performs worse than the other atomic classes and this is consistent with that was observed previously. Additionally, Table 5 also shows the segmentation results for the 3 types of electronegativity maps as average class wise dice coefficient values over the test set.

Table 4: Metrics for the joint learning of molecular structure and the prediction of electronegativity maps. The average metrics obtained are similar to the singular structure prediction task. However, see atomic class 6, silicon being the least accurately predicted class again.

| Atomic Number | Atomic Class Number | ROP | L1 error | Dice |
|---|---|---|---|---|
| - | 0 | 0.9985 | - | 0.9992 |
| 1 | 1 | 2.3200 | 32.5635 | 0.5572 |
| 6 | 2 | 5.6698 | 67.3922 | 0.3048 |
| 7 | 3 | 1.8221 | 3.4230 | 0.4624 |
| 8 | 4 | 2.9951 | 7.9437 | 0.5254 |
| 9 | 5 | 3.5512 | 24.5 | 0.2135 |

(continued)

| Atomic Number | Atomic Class Number | ROP | L1 error | Dice |
|---|---|---|---|---|
| 14 | 6 | 8.1500 | 10.25 | 0.04598 |
| 15 | 7 | 1.3333 | 4.6667 | 0.1333 |
| 16 | 8 | 1.3750 | 2.75 | 0.3055 |
| 17 | 9 | 2.1530 | 10.1999 | 0.6786 |
| 35 | 10 | 2.3333 | 2.0 | 0.6888 |
| ∅ | - | 2.7777 | 16.5689 | 0.4467 |

*Table 5: Dice coefficients values were used to evaluate the overlap of the electronegativity map ground truth to the prediction. Atomic class 1 consisted of the low reactive sites, atomic class 2 consisted of the high reactive sites and atomic class 0 are all the other voxels. The atomic class imbalance between the three atomic classes can be also seen in the results with low dice coefficient for the minority classes.*

| Atomic Class Number | Dice Coef |
|---|---|
| 0 | 0.9479 |
| 1 | 0.2567 |
| 2 | 0.3889 |

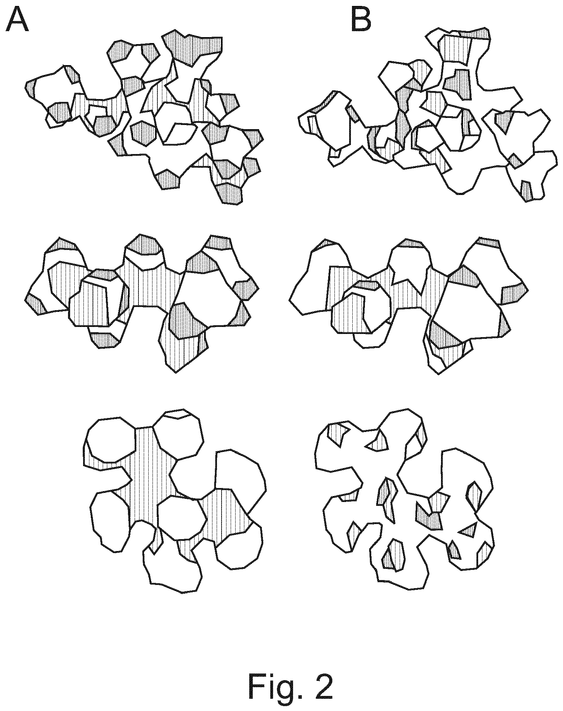

To visually inspect the results of the electronegativity map calculation, three molecules were chosen randomly from the test set and their predicted electronegativity maps were overlaid on their 3D electron density structures. These are shown in **figure 2** with the ground truths shown on the left (A) and the calculated regions shown on the right (B). The SMILES strings of the illustrated molecules are CCOC1(CCC2C(=C)C1CCC2(C)C)C, CC(CC(C)(C)O)O, and [C@@H]([C@H](C(=O)O)O)(C(=O)O)O respectively. The shaded, black region with white stripes and white colored regions are those marked as centers of high, low and medium electronegativity respectively, with the same interpretation as in figure 1.

Visually, the segmentation results also show what was observed in the average dice coefficients shown in Table 5. While there seems to be an overlap between the ground truth voxels and their predicted segmentation results, however, the electronegativity class imbalance between the medium reactive sites and the other two electronegativity classes can also be seen especially around the 'edges' of those high and low reactive regions.

**Example 3** - **Classification**

A test dataset of overall 855 substances from the 'Allowed/Non-Hazardous' class, and 501 molecules from the 'Prohibited/Health Hazardous' class that can be also considered as toxic for cosmetic usage was given. After training for 50 epochs with a learning rate of 1e-3 and rate decay after 25 epochs and a weight decay of 1e-6 on Adam optimizer, an accuracy of 67.75% on the test set was achieved denoting that this strategy can be used to classify substances into health hazardous and non-hazardous classes.

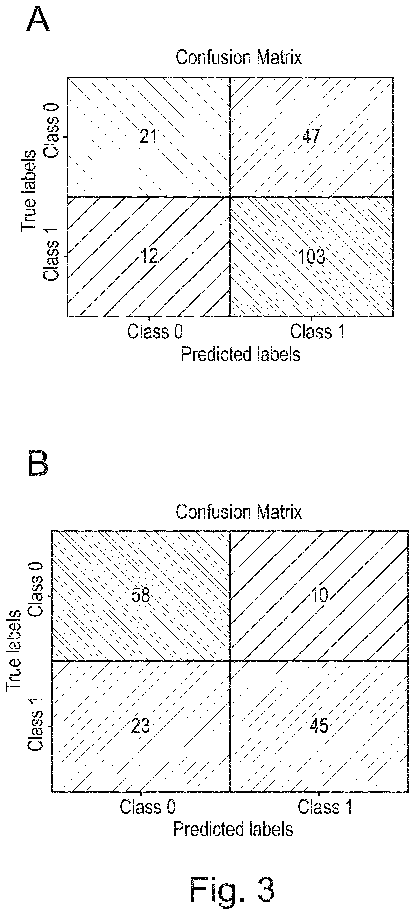

The corresponding confusion matrix is shown in **figure 3 (A).** According to the purpose of the invention one is more interested in not only the overall accuracy but also in the false negatives for class 0, i.e., substances originally marked as not allowed or health hazardous in the data but predicted as allowed.

To combat the class imbalance, in a further step a class weight in the binary cross entropy loss equal to the inverse ratio of occurrence of the two classes was introduced. The same setup as already described was tested on the balanced dataset, i.e., where the number of samples for both classes were identical across all subsets, train, test and validation sets. To create a balanced dataset, the majority class was undersampled. The balanced dataset distribution is shown in Table 6. For this purpose, the same hyperparameters as for the unbalanced data were used.

*Table 6: Distribution of the two classes across for balanced dataset.*

| Class $C_i$ | Class Index $i$ | Train | Validation | Test |
|---|---|---|---|---|
| Prohibited/Health Hazardous | 0 | 501 | 122 | 68 |

(continued)

| Class $C_i$ | Class Index $i$ | Train | Validation | Test |
|---|---|---|---|---|
| Allowed/Non-Hazardous | 1 | 501 | 122 | 68 |
| Total | | 1002 | 244 | 136 |

The confusion matrix for classification on the balanced dataset illustrated in **figure 3 (B)** shows that the classification improved on the balanced dataset., especially fewer substances marked originally as prohibited/heath hazardous were predicted as allowed/non-hazardous. The confusion matrix shown in figure 3 (B) shows that balancing the dataset helped in improving our accuracy to 75.73% and this also reduced the false negative rate in the predictions.

**References**

[1] a) L. G. Fine, C. E. Riera, Frontiers in physiology 2019, 10, 1151; b) P. Morquecho-Campos, K. de Graaf, S. Boesveldt, Food quality and preference 2020, 85, 103959.

[2] J. E. Taylor, H. Lau, B. Seymour, A. Nakae, H. Sumioka, M. Kawato, A. Koizumi, Frontiers in Neuroscience 2020, 14, 255.

[3] Heller, S. R.; McNaught, A.; Pletnev, I.; Stein, S.; Tchekhovskoi, D. InChl, the IUPAC International Chemical Identifier. J. Cheminform.

[4] Anderson, E.; Veith, G. D.; Weininger, D. SMILES: A Line Notation and Computerized Interpreter for Chemical Structures.; 1987.

[5] Daylight Theory: SMARTS - A Language for Describing Molecular Patterns. 2012, pp 1-7.

[6] Jin, W.; Barzilay, R.; Jaakkola, T. Chapter 11: Junction Tree Variational Autoencoder for Molecular Graph Generation. RSC Drug Discov. Ser. 2021, 2021-Janua (75), 228-249. https://doi.org/10.1039/9781788016841-00228.

[7] Takeda, S.; Hama, T.; Hsu, H.-H.; Yamane, T.; Masuda, K.; Piunova, V. A.; Zubarev, D.; Pitera, J.; Sanders, D. P.; Nakano, D. AI-Driven Inverse Design System for Organic Molecules. 2020.

[8] De Cao, N.; Kipf, T. MolGAN: An Implicit Generative Model for Small Molecular Graphs. 2018.

[9] Gómez-Bombarelli, R.; Wei, J. N.; Duvenaud, D.; Hernández-Lobato, J. M.; Sánchez-Lengeling, B.; Sheberla, D.; Aguilera-Iparraguirre, J.; Hirzel, T. D.; Adams, R. P.; Aspuru-Guzik, A. Automatic Chemical Design Using a Data-Driven Continuous Representation of Molecules. ACS Cent. Sci. 2018, 4 (2), 268-276. https://doi.org/10.1021/acscentsci.7b00572.

[10] Méndez-Lucio, O.; Baillif, B.; Clevert, D.-A.; Rouquié, D.; Wichard, J. De Novo Generation of Hit-like Molecules from Gene Expression Signatures Using Artificial Intelligence. Nat. Commun. 2020, 11 (1), 10. https://doi.org/10.1038/s41467-019-13807-w.

[11] Skalic, M.; Jiménez, J.; Sabbadin, D.; De Fabritiis, G. Shape-Based Generative Modeling for de Novo Drug Design. J. Chem. Inf. Model. 2019, 59 (3), 1205-1214. https://doi.org/10.1021/acs.jcim.8b00706.

[12] Bjerrum, E. J.; Threlfall, R. Molecular Generation with Recurrent Neural Networks (RNNs). 2017.

[13] Jastrzębski, S.; Lesniak, D.; Czarnecki, W. M. Learning to SMILE(S). 2016, 1-5.

[14] Arús-Pous, J.; Patronov, A.; Bjerrum, E. J.; Tyrchan, C.; Reymond, J. L.; Chen, H.; Engkvist, O. SMILES-Based Deep Generative Scaffold Decorator for de-Novo Drug Design. J. Cheminform. 2020, 12 (1), 1-32. https://doi.org/10.1186/s13321-020-00441-8.

[15] Zhavoronkov, A.; Ivanenkov, Y. A.; Aliper, A.; Veselov, M. S.; Aladinskiy, V. A.; Aladinskaya, A. V; Terentiev, V. A.; Polykovskiy, D. A.; Kuznetsov, M. D.; Asadulaev, A.; Volkov, Y.; Zholus, A.; Shayakhmetov, R. R.; Zhebrak, A.; Minaeva, L. I.; Zagribelnyy, B. A.; Lee, L. H.; Soll, R.; Madge, D.; Xing, L.; Guo, T.; Aspuru-Guzik, A. Deep Learning Enables Rapid Identification of Potent DDR1 Kinase Inhibitors. Nat. Biotechnol. 2019, 37 (9), 1038-1040. https://doi.org/10.1038/s41587-019-0224-x.

[16] Popova, M.; Isayev, O.; Tropsha, A. Deep Reinforcement Learning for de Novo Drug Design. Sci. Adv. 2022, 4 (7),

[17] Liu, Q.; Allamanis, M.; Brockschmidt, M.; Gaunt, A. L. Constrained Graph Variational Autoencoders for Molecule Design. Adv. Neural Inf. Process. Syst. 2018, 2018-Decem (NeurIPS), 7795-7804.

[18] Li, Y.; Vinyals, O.; Dyer, C.; Pascanu, R.; Battaglia, P. Learning Deep Generative Models of Graphs. 2018.

[19] You, J.; Ying, R.; Ren, X.; Hamilton, W. L.; Leskovec, J. GraphRNN: Generating Realistic Graphs with Deep Auto-Regressive Models. 35th Int. Conf. Mach. Learn. ICML 2018 2018, 13, 9072-9081.

[20] Ma, T.; Chen, J.; Xiao, C. Constrained Generation of Semantically Valid Graphs via Regularizing Variational Autoencoders. Adv. Neural Inf. Process. Syst. 2018, 2018-Decem (Nips), 7113-7124.

[21] Shervani-Tabar, N.; Zabaras, N. Physics-Constrained Predictive Molecular Latent Space Discovery with Graph Scattering Variational Autoencoder. arXiv 2020, 1-42.

[22] Walters, W. P.; Barzilay, R. Applications of Deep Learning in Molecule Generation and Molecular Property Prediction. Acc. Chem. Res. 2021, 54 (2), 263-270. https://doi.org/10.1021/acs.accounts.0c00699.

[23] Hirohara, M.; Saito, Y.; Koda, Y.; Sato, K.; Sakakibara, Y. Convolutional Neural Network Based on SMILES Representation of Compounds for Detecting Chemical Motif. BMC Bioinformatics 2018, 19 (19), 526. https://doi.org/10.1186/s12859-018-2523-5.

[24] Mayr, A.; Klambauer, G.; Unterthiner, T.; Hochreiter, S. DeepTox: Toxicity Prediction Using Deep Learning. Front. Environ. Sci. 2016, 3 (FEB). https://doi.org/10.3389/fenvs.2015.00080.

[25] Suzuki, T.; Katouda, M. Predicting Toxicity by Quantum Machine Learning. J. Phys. Commun. 2020, 4 (12), 1-30. https://doi.org/10.1088/2399-6528/abd3d8.

[26] Sanchez-Lengeling, B.; Wei, J. N.; Lee, B. K.; Gerkin, R. C.; Aspuru-Guzik, A.; Wiltschko, A. B. Machine Learning for Scent: Learning Generalizable Perceptual Representations of Small Molecules. 2019.

[27] Keller, A.; Gerkin, R. C.; Guan, Y.; Dhurandhar, A.; Turu, G.; Szalai, B.; Mainland, J. D.; Ihara, Y.; Yu, C. W.; Wolfinger, R.; Vens, C.; Schietgat, L.; De Grave, K.; Norel, R.; Stolovitzky, G.; Cecchi, G. A.; Vosshall, L. B.; Meyer, P.; Bhondekar, A. P.; Boutros, P. C.; Chang, Y. C.; Chen, C. Y.; Cherng, B. W.; Dimitriev, A.; Dolenc, A.; Falcao, A. O.; Golinska, A. K.; Hong, M. Y.; Hsieh, P. H.; Huang, B. F.; Hunyady, L.; Kaur, R.; Kazanov, M. D.; Kumar, R.; Lesinski, W.; Lin, X.; Matteson, A.; Oyang, Y. J.; Panwar, B.; Piliszek, R.; Polewko-Klim, A.; Raghava, G. P. S.; Rudnicki, W. R.; Saiz, L.; Sun, R. X.; Toplak, M.; Tung, Y. A.; Us, P.; Várnai, P.; Vilar, J.; Xie, M.; Yao, D.; Zitnik, M.; Zupan, B. Predicting Human Olfactory Perception from Chemical Features of Odor Molecules. Science (80). 2017, 355 (6327), 820-826. https://doi.org/10.1126/science.aal2014.

[28] Genva, M.; Kemene, T. K.; Deleu, M.; Lins, L.; Fauconnier, M. L. Is It Possible to Predict the Odor of a Molecule on the Basis of Its Structure? Int. J. Mol. Sci. 2019, 20 (12). https://doi.org/10.3390/ijms20123018.

[29] Lötsch, J.; Kringel, D.; Hummel, T. Machine Learning in Human Olfactory Research. Chem. Senses 2019, 44 (1), 11-22. https://doi.org/10.1093/chemse/bjy067.

[30] Shang, L.; Liu, C.; Tomiura, Y.; Hayashi, K. Machine-Learning-Based Olfactometer: Prediction of Odor Perception from Physicochemical Features of Odorant Molecules. Anal. Chem. 2017, 89 (22), 11999-12005. https://doi.org/10.1021/acs.analchem.7b02389.

[31] Cangea, C.; Grauslys, A.; Liò, P.; Falciani, F. Structure-Based Networks for Drug Validation. 2018, 1-5.

[32] Sakai, M.; Nagayasu, K.; Shibui, N.; Andoh, C.; Takayama, K.; Shirakawa, H.; Kaneko, S. Prediction of Pharmacological Activities from Chemical Structures with Graph Convolutional Neural Networks. Sci. Rep. 2021, 11 (1), 525. https://doi.org/10.1038/s41598-020-80113-7.

[33] Gaudelet, T.; Day, B.; Jamasb, A. R.; Soman, J.; Regep, C.; Liu, G.; Hayter, J. B. R.; Vickers, R.; Roberts, C.; Tang, J.; Roblin, D.; Blundell, T. L.; Bronstein, M. M.; Taylor-King, J. P. Utilizing Graph Machine Learning within Drug Discovery and Development. Brief. Bioinform. 2021, 22 (6), bbab159. https://doi.org/10.1093/bib/bbab159.

[34] Jiang, D.; Wu, Z.; Hsieh, C.-Y.; Chen, G.; Liao, B.; Wang, Z.; Shen, C.; Cao, D.; Wu, J.; Hou, T. Could Graph Neural Networks Learn Better Molecular Representation for Drug Discovery? A Comparison Study of Descriptor-Based and Graph-Based Models. J. Cheminform. 2021, 13 (1), 12. https://doi.org/10.1186/s13321-020-00479-8.

[35] David, L.; Thakkar, A.; Mercado, R.; Engkvist, O. Molecular Representations in AI-Driven Drug Discovery: A Review and Practical Guide. J. Cheminform. 2020, 12 (1), 1-22. https://doi.org/10.1186/s13321-020-00460-5.

[36] O'Boyle, N. M. Towards a Universal SMILES Representation - A Standard Method to Generate Canonical SMILES Based on the InChl. J. Cheminform. 2012, 4 (1), 22. https://doi.org/10.1186/1758-2946-4-22.

[37] D. Weininger, Journal of chemical information and computer sciences 1988, 28, 31.

[38] Daylight Chemical Information Systems, Inc., "3. SMILES - A Simplified Chemical Language", can be found under https://www.daylight.com/dayhtml/doc/theory/theory.smiles.html, 2019.

[39] Daylight Chemical Information Systems, Inc., "4. SMARTS - A Language for Describing Molecular Patterns", can be found under https://www.daylight.com/dayhtml/doc/theory/theory.smarts.html, 2019.

[40] Halgren, T. A. Performance of MMFF94*. Scope, Parameterization, J. Comput. Chem. 1996, 17, 490-519.

[41] RDKit: Open-Source Cheminformatics. http://www.rdkit.org/.

[42] Empire & EH5cube. https://www.ceposinsilico.de/products/empire.htm.

[43] Dewar, M. J. S.; Zoebisch, E. G.; Healy, E. F.; Stewart, J. J. P. Development and Use of Quantum Mechanical Molecular Models. 76. AM 1: A New General Purpose Quantum Mechanical Molecular Model. J. Am. Chem. Soc. 1985, 107 (13), 3902-3909. https://doi.org/10.1021/ja00299a024.

[44] Gaussian 16: https://gaussian.com/gaussian16/ ; https://gaussian.com/dft/

[45] Nordholm, S. From Electronegativity towards Reactivity-Searching for a Measure of Atomic Reactivity. Molecules 2021, 26 (12). https://doi.org/10.3390/molecules26123680.

[46] Lewis, A. M.; Grisafi, A.; Ceriotti, M.; Rossi, M. Learning Electron Densities in the Condensed Phase. J. Chem. Theory Comput. 2021, 17 (11), 7203-7214. https://doi.org/10.1021/acs.jctc.1c00576.

[47] Parr, R. G.; Weitao, Y. Density-Functional Theory of Atoms and Molecules. Oxford University Press January 5, 1995. https://doi.org/10.1093/oso/9780195092769.001.0001.

[48] Lovering, F.; Bikker, J.; Humblet, C. Escape from Flatland: Increasing Saturation as an Approach to Improving Clinical Success. J. Med. Chem. 2009, 52 (21), 6752-6756. https://doi.org/10.1021/jm901241e.

[49] Méndez-Lucio, O.; Medina-Franco, J. L. The Many Roles of Molecular Complexity in Drug Discovery. Drug Discov. Today 2017, 22 (1), 120-126. https://doi.org/10.1016/j.drudis.2016.08.009.

[50] Paszke, A.; Gross, S.; Massa, F.; Lerer, A.; Bradbury, J.; Chanan, G.; Killeen, T.; Lin, Z.; Gimelshein, N.; Antiga, L.; Desmaison, A.; Köpf, A.; Yang, E.; DeVito, Z.; Raison, M.; Tejani, A.; Chilamkurthy, S.; Steiner, B.; Fang, L.; Bai, J.; Chintala, S. PyTorch: An Imperative Style, High-Performance Deep Learning Library. Adv. Neural Inf. Process. Syst. 2019, 32 (NeurIPS).

[51] Sudre, C. H.; Li, W.; Vercauteren, T.; Ourselin, S.; Jorge Cardoso, M. Generalised Dice Overlap as a Deep Learning Loss Function for Highly Unbalanced Segmentations. Lect. Notes Comput. Sci. (including Subser. Lect. Notes Artif. Intell. Lect. Notes Bioinformatics) 2017, 10553 LNCS, 240-248. https://doi.org/10.1007/978-3-319-67558-9_28.

## Claims

1. Method for screening substances having a required physical property, chemical property or physiological effect from a group of substances comprising the steps

   • providing a group of $k$ substances by a user, wherein $k \in N$;
   • providing a classification or a property label for a physical property, chemical property or physiological effect comprising $C_i$ classes or $V_i$ values, wherein $i \in N$;
   • calculating electron density cube files for every substance by semiempirical molecular calculation and providing weights $W_k$, such as electronegativity or electron affinity cube files for every substance;
   • providing a trained artificial neural network architecture for the classification or label property, wherein the artificial neural network architecture uses the electron density cube files and weights $W_k$ as input to calculate a tensor of shape of each substance describing the spatial structure of the substance and a tensor of weights, such as electronegativity or electron affinity assigned to the spatial structure of the substance;
   • assigning each substance to a class $C_i$ or a value $V_i$ by utilizing the tensor of weights, such as electronegativity or electron affinity of substance k as weighting for the electronic density cube files in the artificial neural network architecture;
   • displaying and/or outputting of the substances assigned to the classes $C_i$ of the classification or assigned to the values $V_i$ of the property label;
   • selecting the substances assigned to the class $C_i$ with the required physical property, chemical property or physiological effect or assigned to the required value Vi of a physical property, chemical property or physiological effect;
   • experimental verification by a user of the physical property, chemical property or physiological effect of at least part of the selected substances.

2. Method according to claim 1, **characterized in that** the trained artificial neural network architecture for the classification or property label is trained for a defined classification or property label by a training dataset, wherein the training dataset consists of substances with known assignment to the classes $C_i$ of the classification or with known assignments to the value $V_i$ of the property label.

3. Method according to one of the preceding claims, **characterized in that** artificial neural network architecture is a segmentation network or a Generative Adversarial Network (GAN).

4. Method according to claim 3, **characterized in that** the segmentation network is modified 3D-UNet, VNet or Inception architecture, or similar networks.

5. Method according to one of the preceding claims, **characterized in that** the tensor of electronegativity comprises the spatial distribution of the electronegativity encoded in discrete electronegativity classes.

6. Method according to claim 4, **characterized in that** the electronegativity is encoded in two electronegativity classes or in three electronegativity classes.

7. Method according to one of the preceding claims, **characterized in that** the electronic density cube file and their corresponding local electronegativity map is illustrated as a substance structure with an overlaid electronegativity map.

8. Method according to one of the preceding claims, **characterized in that** the classification or property label is selected from the group of structure-based properties of molecules, in particular from the group containing odour, taste, colour, water solubility, volatility, toxicity, permitted and non-permitted chemicals in cosmetics and personal care, boiling point, melting point, solubility, aroma thresholds, partition coefficient.

9. Use of the method according to claim 1 to 8 to select at least one substance with a requested chemical property, physical property or physiological effect from a group of substances.

A

B

Fig. 1

A    B

Fig. 2

A

Confusion Matrix

B

Confusion Matrix

Fig. 3

Fig. 4

Europäisches
Patentamt

European
Patent Office

Office européen
des brevets

## EUROPEAN SEARCH REPORT

Application Number

EP 23 16 1477

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YONG ZHAO ET AL: "Predicting Elastic Properties of Materials from Electronic Charge Density Using 3D Deep Convolutional Neural Networks", ARXIV.ORG, CORNELL UNIVERSITY LIBRARY, 201 OLIN LIBRARY CORNELL UNIVERSITY ITHACA, NY 14853, 17 March 2020 (2020-03-17), XP081631608, * the whole document * | 1-9 | INV. G16C20/30 G16C20/64 |
| A | JÖRN LÖTSCH ET AL: "Machine Learning in Human Olfactory Research", CHEMICAL SENSES, vol. 44, no. 1, 27 October 2018 (2018-10-27), pages 11-22, XP055711692, GB ISSN: 0379-864X, DOI: 10.1093/chemse/bjy067 * the whole document * | 1-9 | |

TECHNICAL FIELDS
SEARCHED     (IPC)

G16C

The present search report has been drawn up for all claims

| Place of search | Date of completion of the search | Examiner |
|---|---|---|
| The Hague | 25 August 2023 | Nurmi, Jussi |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. FINE ; C. E. RIERA.** *Frontiers in physiology,* 2019, vol. 10, 1151
- **P. MORQUECHO-CAMPOS ; K. DE GRAAF ; S. BOESVELDT.** *Food quality and preference,* 2020, vol. 85, 103959
- **J. E. TAYLOR ; H. LAU ; B. SEYMOUR ; A. NAKAE ; H. SUMIOKA ; M. KAWATO ; A. KOIZU-MI.** *Frontiers in Neuroscience,* 2020, vol. 14, 255
- **HELLER, S. R. ; MCNAUGHT, A. ; PLETNEV, I. ; STEIN, S. ; TCHEKHOVSKOI, D.** InChI, the IUPAC International Chemical Identifier. *J. Cheminform.*
- **ANDERSON, E. ; VEITH, G. D. ; WEININGER, D.** *SMILES: A Line Notation and Computerized Interpreter for Chemical Structures.,* 1987
- *Daylight Theory: SMARTS - A Language for Describing Molecular Patterns,* 2012, 1-7
- Junction Tree Variational Autoencoder for Molecular Graph Generation. **JIN, W. ; BARZILAY, R. ; JAAK-KOLA, T.** RSC Drug Discov. Ser. 2021. January 2021, 228-249
- **TAKEDA, S. ; HAMA, T. ; HSU, H.-H. ; YAMANE, T. ; MASUDA, K. ; PIUNOVA, V. A. ; ZUBAREV, D. ; PITERA, J. ; SANDERS, D. P. ; NAKANO, D.** *AI-Driven Inverse Design System for Organic Molecules,* 2020
- **DE CAO, N. ; KIPF, T.** *MolGAN: An Implicit Generative Model for Small Molecular Graphs,* 2018
- **GÓMEZ-BOMBARELLI, R. ; WEI, J. N. ; DUVE-NAUD, D. ; HERNÁNDEZ-LOBATO, J. M. ; SÁNCHEZ-LENGELING, B. ; SHEBERLA, D. ; AGUILERA-LPARRAGUIRRE, J. ; HIRZEL, T. D. ; ADAMS, R. P. ; ASPURU-GUZIK, A.** Automatic Chemical Design Using a Data-Driven Continuous Representation of Molecules. *ACS Cent. Sci.,* 2018, vol. 4 (2), 268-276, https://doi.org/10.1021/acscentsci.7b00572
- **MÉNDEZ-LUCIO, O. ; BAILLIF, B. ; CLEVERT, D.-A. ; ROUQUIÉ, D. ; WICHARD, J.** De Novo Generation of Hit-like Molecules from Gene Expression Signatures Using Artificial Intelligence. *Nat. Commun.,* 2020, vol. 11 (1), 10, https://doi.org/10.1038/s41467-019-13807-w
- **SKALIC, M. ; JIMÉNEZ, J. ; SABBADIN, D. ; DE FABRITIIS, G.** Shape-Based Generative Modeling for de Novo Drug Design. *J. Chem. Inf. Model.,* 2019, vol. 59 (3), 1205-1214, https://doi.org/10.1021/acs.jcim.8b00706

- **BJERRUM, E. J. ; THRELFALL, R.** *Molecular Generation with Recurrent Neural Networks (RNNs),* 2017
- **ARÚS-POUS, J. ; PATRONOV, A. ; BJERRUM, E. J. ; TYRCHAN, C. ; REYMOND, J. L. ; CHEN, H. ; ENGKVIST, O.** SMILES-Based Deep Generative Scaffold Decorator for de-Novo Drug Design. *J. Cheminform.,* 2020, vol. 12 (1), 1-32, https://doi.org/10.1186/s13321-020-00441-8
- **ZHAVORONKOV, A. ; IVANENKOV, Y. A. ; ALI-PER, A. ; VESELOV, M. S. ; ALADINSKIY, V. A. ; ALADINSKAYA, A. V ; TERENTIEV, V. A. ; POLYK-OVSKIY, D. A. ; KUZNETSOV, M. D. ; ASADU-LAEV, A.** Deep Learning Enables Rapid Identification of Potent DDR1 Kinase Inhibitors. *Nat. Biotechnol.,* 2019, vol. 37 (9), 1038-1040, https://doi.org/10.1038/s41587-019-0224-x
- **POPOVA, M. ; ISAYEV, O. ; TROPSHA, A.** Deep Reinforcement Learning for de Novo Drug Design. *Sci. Adv.,* 2022, vol. 4 (7
- **LIU, Q. ; ALLAMANIS, M. ; BROCKSCHMIDT, M. ; GAUNT, A. L.** Constrained Graph Variational Autoencoders for Molecule Design. *Adv. Neural Inf. Process. Syst.,* December 2018, 7795-7804
- **LI, Y. ; VINYALS, O. ; DYER, C. ; PASCANU, R. ; BATTAGLIA, P.** *Learning Deep Generative Models of Graphs,* 2018
- **YOU, J. ; YING, R. ; REN, X. ; HAMILTON, W. L. ; LESKOVEC, J.** GraphRNN: Generating Realistic Graphs with Deep Auto-Regressive Models. *35th Int. Conf. Mach. Learn. ICML,* 2018, vol. 2018 (13), 9072-9081
- **MA, T. ; CHEN, J. ; XIAO, C.** Constrained Generation of Semantically Valid Graphs via Regularizing Variational Autoencoders. *Adv. Neural Inf. Process. Syst.,* December 2018, 7113-7124
- **SHERVANI-TABAR, N. ; ZABARAS, N.** Physics-Constrained Predictive Molecular Latent Space Discovery with Graph Scattering Variational Autoencoder. *arXiv,* 2020, 1-42
- **WALTERS, W. P. ; BARZILAY, R.** Applications of Deep Learning in Molecule Generation and Molecular Property Prediction. *Acc. Chem. Res.,* 2021, vol. 54 (2), 263-270, https://doi.org/10.1021/acs.accounts.0c00699

- **HIROHARA, M. ; SAITO, Y. ; KODA, Y. ; SATO, K. ; SAKAKIBARA, Y.** Convolutional Neural Network Based on SMILES Representation of Compounds for Detecting Chemical Motif. *BMC Bioinformatics,* 2018, vol. 19 (19), 526, https://doi.org/10.1186/s12859-018-2523-5
- **MAYR, A. ; KLAMBAUER, G. ; UNTERTHINER, T. ; HOCHREITER, S.** DeepTox: Toxicity Prediction Using Deep Learning. *Front. Environ. Sci.,* February 2016, 3, https://doi.org/10.3389/fenvs.2015.00080
- **SUZUKI, T. ; KATOUDA, M.** Predicting Toxicity by Quantum Machine Learning. *J. Phys. Commun.,* 2020, vol. 4 (12), 1-30, https://doi.org/10.1088/2399-6528/abd3d8
- **SANCHEZ-LENGELING, B. ; WEI, J. N. ; LEE, B. K. ; GERKIN, R. C. ; ASPURU-GUZIK, A. ; WILTSCHKO, A. B.** *Machine Learning for Scent: Learning Generalizable Perceptual Representations of Small Molecules,* 2019
- **GENVA, M. ; KEMENE, T. K. ; DELEU, M. ; LINS, L. ; FAUCONNIER, M. L.** Is It Possible to Predict the Odor of a Molecule on the Basis of Its Structure?. *Int. J. Mol. Sci.,* 2019, vol. 20 (12, https://doi.org/10.3390/ijms20123018
- **LÖTSCH, J. ; KRINGEL, D. ; HUMMEL, T.** Machine Learning in Human Olfactory Research. *Chem. Senses,* 2019, vol. 44 (1), 11-22, https://doi.org/10.1093/chemse/bjy067
- **SHANG, L. ; LIU, C. ; TOMIURA, Y. ; HAYASHI, K.** Machine-Learning-Based Olfactometer: Prediction of Odor Perception from Physicochemical Features of Odorant Molecules. *Anal. Chem.,* 2017, vol. 89 (22), 11999-12005, https://doi.org/10.1021/acs.analchem.7b02389
- **CANGEA, C. ; GRAUSLYS, A. ; LIÒ, P. ; FALCIANI, F.** *Structure-Based Networks for Drug Validation,* 2018, 1-5
- **SAKAI, M. ; NAGAYASU, K. ; SHIBUI, N. ; ANDOH, C. ; TAKAYAMA, K. ; SHIRAKAWA, H. ; KANEKO, S.** Prediction of Pharmacological Activities from Chemical Structures with Graph Convolutional Neural Networks. *Sci. Rep.,* 2021, vol. 11 (1), 525, https://doi.org/10.1038/s41598-020-80113-7
- **GAUDELET, T. ; DAY, B. ; JAMASB, A. R. ; SOMAN, J. ; REGEP, C. ; LIU, G. ; HAYTER, J. B. R. ; VICKERS, R. ; ROBERTS, C. ; TANG, J.** Utilizing Graph Machine Learning within Drug Discovery and Development. *Brief. Bioinform.,* 2021, vol. 22 (6), 159, https://doi.org/10.1093/bib/bbab159
- **JIANG, D. ; WU, Z. ; HSIEH, C.-Y. ; CHEN, G. ; LIAO, B. ; WANG, Z. ; SHEN, C. ; CAO, D. ; WU, J. ; HOU, T.** Could Graph Neural Networks Learn Better Molecular Representation for Drug Discovery? A Comparison Study of Descriptor-Based and Graph-Based Models. *J. Cheminform.,* 2021, vol. 13 (1), 12, https://doi.org/10.1186/s13321-020-00479-8
- **DAVID, L. ; THAKKAR, A. ; MERCADO, R. ; ENGKVIST, O.** Molecular Representations in AI-Driven Drug Discovery: A Review and Practical Guide. *J. Cheminform.,* 2020, vol. 12 (1), 1-22, https://doi.org/10.1186/s13321-020-00460-5
- **O'BOYLE, N. M.** Towards a Universal SMILES Representation - A Standard Method to Generate Canonical SMILES Based on the InChI. *J. Cheminform.,* 2012, vol. 4 (1), 22, https://doi.org/10.1186/1758-2946-4-22
- **D. WEININGER.** *Journal of chemical information and computer sciences,* 1988, vol. 28, 31
- 3. SMILES - A Simplified Chemical Language. Daylight Chemical Information Systems, Inc, 2019
- 4. SMARTS - A Language for Describing Molecular Patterns. Daylight Chemical Information Systems, Inc, 2019
- **HALGREN, T. A.** Performance of MMFF94*. Scope, Parameterization. *J. Comput. Chem.,* 1996, vol. 17, 490-519
- *RDKit: Open-Source Cheminformatics,* http://www.rdkit.org
- *Empire & EH5cube,* https://www.ceposinsilico.de/products/empire.htm
- **DEWAR, M. J. S. ; ZOEBISCH, E. G. ; HEALY, E. F. ; STEWART, J. J. P.** Development and Use of Quantum Mechanical Molecular Models. 76. AM 1: A New General Purpose Quantum Mechanical Molecular Model. *J. Am. Chem. Soc.,* 1985, vol. 107 (13), 3902-3909, https://doi.org/10.1021/ja00299a024
- *Gaussian 16,* https://gaussian.com/gaussian16
- **NORDHOLM, S.** From Electronegativity towards Reactivity-Searching for a Measure of Atomic Reactivity. *Molecules,* 2021, vol. 26 (12, https://doi.org/10.3390/molecules26123680
- **LEWIS, A. M. ; GRISAFI, A. ; CERIOTTI, M. ; ROSSI, M.** Learning Electron Densities in the Condensed Phase. *J. Chem. Theory Comput.,* 2021, vol. 17 (11), 7203-7214, https://doi.org/10.1021/acs.jctc.1c00576
- **PARR, R. G. ; WEITAO, Y.** Density-Functional Theory of Atoms and Molecules. Oxford University Press, 05 January 1995
- **LOVERING, F. ; BIKKER, J. ; HUMBLET, C.** Escape from Flatland: Increasing Saturation as an Approach to Improving Clinical Success. *J. Med. Chem.,* 2009, vol. 52 (21), 6752-6756, https://doi.org/10.1021/jm901241e
- **MÉNDEZ-LUCIO, O. ; MEDINA-FRANCO, J. L.** The Many Roles of Molecular Complexity in Drug Discovery. *Drug Discov. Today,* 2017, vol. 22 (1), 120-126, https://doi.org/10.1016/j.drudis.2016.08.009

- **PASZKE, A. ; GROSS, S. ; MASSA, F. ; LERER, A. ; BRADBURY, J. ; CHANAN, G. ; KILLEEN, T. ; LIN, Z. ; GIMELSHEIN, N. ; ANTIGA, L.** PyTorch: An Imperative Style, High-Performance Deep Learning Library. *Adv. Neural Inf. Process. Syst.,* 2019, 32

- **SUDRE, C. H. ; LI, W. ; VERCAUTEREN, T. ; OURSELIN, S. ; JORGE CARDOSO, M.** Generalised Dice Overlap as a Deep Learning Loss Function for Highly Unbalanced Segmentations. Lect. Notes Comput. Sci. (including Subser. *Lect. Notes Artif. Intell. Lect. Notes Bioinformatics,* 2017, 240-248, https://doi.org/10.1007/978-3-319-67558-9_28